(19) Europäisches Patentamt
European Patent Office
Office européen des brevets

(11) EP 1 277 746 B1

(12) **FASCICULE DE BREVET EUROPEEN**

(45) Date de publication et mention de la délivrance du brevet:
**11.05.2005 Bulletin 2005/19**

(21) Numéro de dépôt: **02291784.3**

(22) Date de dépôt: **16.07.2002**

(51) Int Cl.[7]: **C07D 295/08**, C07D 239/42, A61K 31/496, A61P 9/00, A61P 25/00, A61P 41/00, C07D 295/14, C07D 239/50

(54) **Nouveaux dérivés de N-benzylpiperazine, leur procédé de préparation et les compositions pharmaceutiques qui les contiennent**

N-Benzylpiperazin N-benzylpiperazine, Verbindungen, Verfahren zu deren Herstellung und deren pharmazeutischen Zusammensetzungen

N-benzylpiperazine derivatives, process for their preparation and pharmaceutical compositions thereof

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **16.07.2001 FR 0109454**

(43) Date de publication de la demande:
**22.01.2003 Bulletin 2003/04**

(73) Titulaire: **Les Laboratoires Servier**
**92200 Neuilly-sur-Seine (FR)**

(72) Inventeurs:
- **Labidalle, Serge**
  **31120 Pinsaguel (FR)**
- **Tillement, Jean-Paul**
  **77590 Bois le Roi (FR)**
- **Testa, Bernard**
  **1005 Lausanne (CH)**
- **Cecchelli, Roméo**
  **59650 Villeneuve d'Ascq (FR)**
- **Le Ridant, Alain**
  **92200 Neuilly sur Seine (FR)**
- **Harpey, Catherine**
  **75016 Paris (FR)**
- **Spedding, Michael**
  **78110 Le Vesinet (FR)**
- **Schenker, Esther**
  **75017 Paris (FR)**

(56) Documents cités:
**EP-A- 0 617 027**
**EP-A- 0 847 999**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés de N-benzylpipérazine, leur procédé de préparation ainsi que les compositions pharmaceutiques qui les contiennent.

L'état antérieur de la technique est illustré notamment par :

- les brevets français 1 302 958 et 805 M qui concernent respectivement la préparation de N-trialkoxy benzyl pipérazines et l'utilisation comme médicament à action vasodilatatrice de la 2,3,4-triméthoxybenzyl pipérazine,
- les articles d'Hiroshi Ohtaka et coll., Chem. Pharm. Bull., 35, 2774-3275 (1987) et Chem. Pharm. Bull., 37, 11, 2124-3122 (1989) qui mentionnent des dérivés de trimétazidine ayant une activité vasodilatatrice, et la synthèse de 1-[bis(4-fluorophényl)méthyl]-4-(2-hydroxy 3,4-diméthoxybenzyl)pipérazine,
- l'article de Tsunéo Kawashima et coll., J. Pharmacobio-Dyn, 14, 449-459 (1991) relatif à l'isolation et l'identification de nouveaux métabolites du KB-2796 dont entre autres la 1-[bis(4-fluorophényl)méthyl]-4-(2-hydroxy 3,4-diméthoxybenzyl)pipérazine,
- le brevet européen EP 533 579 qui décrit des dérivés de N-benzylpipérazines ayant une activité antihypoxique et antiischémique,
- le brevet européen EP 617 027 qui décrit des dérivés de N-benzylpipérazines utiles pour le traitement des maladies neuronales dues au dysfonctionnement du métabolisme oxydatif.
- enfin, le brevet européen EP 847 999 qui décrit des dérivés de N-benzylpipérazines qui permettent, entre autres, la protection des mitochondries subissant un stress hypoxique et qui sont capables de franchir la barrière hémato-encéphalique.

Les dérivés de la présente invention, outre le fait qu'ils soient nouveaux, présentent une activité pharmacologique et des propriétés thérapeutiques particulièrement innovantes.

Ils permettent, entre autres, la protection des mitochondries subissant un stress hypoxique, le maintien de la synthèse d'ATP dans des organes privés d'oxygène comme la protection d'un coeur isolé placé en situation d'ischémie. Enfin, certains sont capables de franchir la barrière hémato-encéphalique. Ces propriétés les rendent donc utiles pour la prévention ou le traitement de l'ischémie cellulaire aigue et chronique, des accidents ischémiques aigus ou chroniques, cérébraux, cardiaques ou périphériques, suivis ou non de reperfusion, avant toute intervention chirurgicale nécessitant un arrêt transitoire de la circulation sanguine, locale ou générale (garrot, clamp...), pour le traitement des maladies neurodégénératives chroniques (comme les démences séniles, la maladie d'Alzheimer sous-jacente ou établie, le vieillissement cérébral, la sclérose latérale amyotrophique, la sclérose en plaques ou la maladie de Parkinson) ainsi que pour l'amélioration de la conservation des organes destinés à être transplantés et la reprise fonctionnelle des greffons après reperfusion.

D'autre part, les composés de l'invention possèdent une stabilité métabolique nettement accrue par rapport à tous les composés de structures proches précédemment décrits, assurent par là même une meilleure biodisponibilité.

Plus spécifiquement, la présente invention concerne les composés de formule (I):

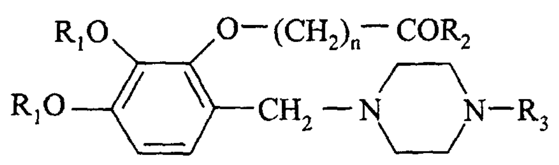

(I)

dans laquelle :

$R_1$ représente un groupement alkyle ($C_1$-$C_6$) linéaire ou ramifié,
$R_2$ représente un groupement hydroxy, alkoxy ($C_1$-$C_6$) linéaire ou ramifié, ou amino (éventuellement substitué par un ou deux groupements alkyle ($C_1$-$C_6$) linéaire ou ramifié),
n représente un nombre entier compris entre 1 et 12 inclusivement.
$R_3$ représente :

- un atome d'hydrogène,
- un groupement alkyle ($C_1$-$C_{12}$) linéaire ou ramifié éventuellement substitué par un groupement phényle (lui-même éventuellement substitué par un ou plusieurs, identiques ou différents, atomes d'halogène ou groupements alkyle ($C_1$-$C_6$) linéaire ou ramifié, alkoxy ($C_1$-$C_6$) linéaire ou ramifié, hydroxy ou trihalogènométhyle), cycloalkyle ($C_3$-$C_7$), carboxy

ou alkoxy ($C_1$-$C_6$)carbonyle,

- un groupement cycloalkyle ($C_3$-$C_7$),
- un groupement pyrimidinyle éventuellement substitué par un ou deux groupements pyrolidinyle,
- ou, un groupement phényle éventuellement substitué par un ou plusieurs, identiques ou différents, atomes d'halogène ou groupements alkyle ($C_1$-$C_6$) linéaire ou ramifié, alkoxy ($C_1$-$C_6$) linéaire ou ramifié, hydroxy ou trihalogènométhyle,

leurs isomères optiques ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I) caractérisé en ce que l'on utilise comme produit de départ un composé de formule (II) :

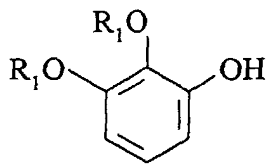

(II)

dans laquelle $R_1$ a la même signification que dans la formule (I), que l'on fait réagir avec une pipérazine substituée de formule (III) :

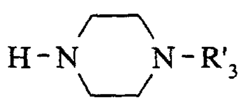

(III)

dans laquelle $R'_3$ a la même signification que $R_3$ à l'exception d'un groupement alkyle substitué par un groupement carboxy, en présence de formaldéhyde, pour conduire au composé de formule (IV) :

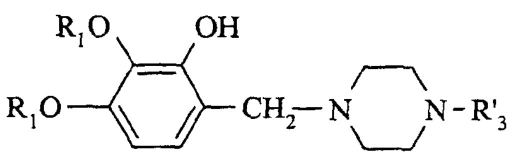

(IV)

dans laquelle $R_1$ et $R'_3$ ont la même signification que précédemment, que l'on fait réagir avec un composé de formule (V) :

$$X\text{-}(CH_2)_n\text{-}COR'_2 \qquad (V)$$

dans laquelle X représente un atome d'halogène et $R'_2$ représente un groupement alkoxy ($C_1$-$C_6$) linéaire ou ramifié, pour conduire au composé de formule (I/a) cas particulier des composés de formule (I) :

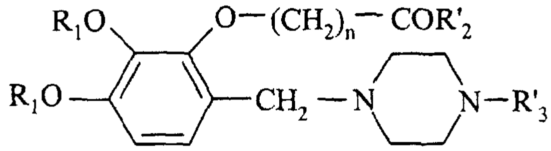

(I/a)

dans laquelle $R_1$, $R'_2$, $R'_3$ et n ont la même signification que précédemment, composé de formule (I/a) que l'on transforme si on le souhaite

- soit en mono ou diacide correspondant de formule (I/b), cas particulier des composés de formule (I) :

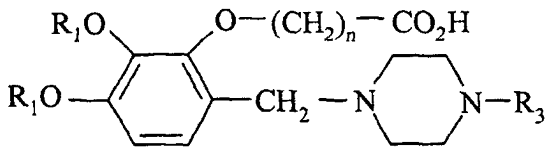

(I/b)

- soit en amide correspondant de formule (I/c):

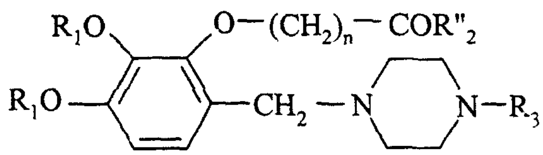

(I/c)

dans laquelle $R_1$, $R_3$ et n ont la même signification que dans la formule (I) et $R''_2$ représente un groupement amino éventuellement substitué par un ou deux groupements alkyle ($C_1$-$C_6$) linéaire ou ramifié,
composés de formule (I/a) (I/b) ou (I/c),
que l'on purifie, le cas échéant, selon une technique classique de purification, dont on sépare éventuellement les isomères, et que l'on transforme, si on le souhaite, en sel d'addition à un acide ou une base pharmaceutiquement acceptable.

Les composés préférés de l'invention sont ceux pour lesquels $R_1$ représente un groupement méthyle, $R_2$ représente un groupement hydroxy et $R_3$ représente un groupement benzyle, un groupement pyrimidinyle, un groupement phényle avantageusement substitué par un groupement méthoxy ou un atome d'hydrogène.

La valeur n est préférentiellement comprise entre 1 et 7. A titre encore plus préférentiel n est égal à 6.

Les composés préférés de l'invention sont :

- l'acide 7-{6-[(4-benzyl-1-pipérazinyl)méthyl]-2,3-diméthoxyphénoxy}heptanoïque ainsi que ses sels d'addition,
- l'acide 7-{6-{[4-(pyrimidin-2-yl)-1-pipéraziny])méthyl}-2,3-diméthoxyphénoxy} heptanoïque ainsi que ses sels d'addition,
- l'acide 4-{6-[(pipérazin-1-yl)méthyl]-2,3-diméthoxyphénoxy}butyrique ainsi que ses sels d'addition,
- l'acide 7-{6-[(4-(2-méthoxyphényl)-1-pipérazinyl)méthyl]-2,3-diméthoxyphénoxy} heptanoïque ainsi que ses sels d'addition,
- l'acide 4-{6-[(4-(2-méthoxyphényl)-1-pipérazinyl)méthyl]-2,3-diméthoxyphénoxy} butyrique ainsi que ses sels d'addition.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, cutanée, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la

voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

***EXEMPLE 1* : 7-{6-[(4-Benzyl-1-pipérazinyl)méthyl]-2,3-diméthoxyphénoxy} heptanoate d'éthyle, dichlorhydrate**

2,2 g d'hydrure de sodium à 60% sont mis en suspension dans un réacteur contenant 250 ml de tétrahydrofurane anhydre refroidit à 0°C. 17,1 g de 6-[(4-benzyl-1-pipérazinyl) méthyl]-2,3-diméthoxyphénol dissous dans 150 ml de THF anhydre sont ajoutés goutte à goutte, à température ambiante au mélange précédent puis 14,6 ml de 7-bromoheptanoate d'éthyle dans 50 ml de THF anhydre.

Le mélange réactionnel est chauffé à reflux pendant 15 jours puis est concentré à sec sous vide partiel. Le résidu brut est repris par l'éther éthylique et le précipité obtenu après refroidissement à 0°C pendant 12h, est filtré, puis lavé par de l'éther éthylique froid (4°C). Les solutions éthérées sont concentrées à sec sous vide partiel et conduisent à un résidu brut qui est purifié par chromatographie sur colonne de silice en utilisant un gradiant de dichlorométhane/méthanol (98/2 à 90/10) pour conduire au produit attendu sous forme de base qui est transformée en dichlorhydrate correspondant.

*Point de fusion* : 180°C

| *Microanalyse élémentaire :* | | | |
|---|---|---|---|
| | **C%** | **H%** | **N%** |
| *Calculée* | *60,94* | *7,76* | *4,90* |
| *Trouvée* | *60,68* | *7,72* | *5,00* |

***EXEMPLE 2* : Acide 7-{6-[(4-benzyl-1-pipérazinyl)méthyl]-2,3-diméthoxyphénoxy} heptanoïque, dichlorhydrate**

10 g du composé obtenu dans l'exemple 1 sont additionnés à une solution contenant 4 g d'hydroxyde de sodium dans 250 ml d'éthanol. L'ensemble est maintenu 6 heures sans agitation. Après évaporation du solvant, le résidu est purifié par chromatographie sur colonne de silice en utilisant un gradiant dichlorométhane/méthanol (95/5 à 0/100).

Le produit attendu est obtenu sous forme de base et transformé en dichlorhydrate correspondant.

*Point de fusion :* 190°C

| *Microanalyse élémentaire :* | | | |
|---|---|---|---|
| | **C%** | **H%** | **N%** |
| *Calculée* | *59,66* | *7,42* | *5,15* |
| *Trouvée* | *59,06* | *7,67* | *5,26* |

***EXEMPLE 3* : {6-[(4-Benzyl-1-pipérazinyl)méthyl]-2,3-diméthoxyphénoxy} acétate d'éthyle, dichlorhydrate**

Le produit attendu est obtenu selon le procédé décrit dans l'exemple 1 en remplaçant le 7-bromoheptanoate d'éthyle par du chloroacétate d'éthyle.

*Point de fusion* : 162°C

| *Microanalyse élémentaire :* | | | |
|---|---|---|---|
| | **C%** | **H%** | **N%** |
| *Calculée* | *57,49* | *6,83* | *5,59* |
| *Trouvée* | *57,62* | *6,87* | *5,62* |

***EXEMPLE 4** : Acide {6-[(4-benzyl-1-pipérazinyl)méthyl]-2,3-diméthoxyphénoxy} acétique, dichlorhydrate*

Le produit attendu est obtenu selon le procédé décrit dans l'exemple 2 à partir du composé décrit dans l'exemple 3.
*Point de fusion* : 175°C

| Microanalyse élémentaire : | | | |
|---|---|---|---|
| | C% | H% | N% |
| Calculée | 55,82 | 6,39 | 5,92 |
| Trouvée | 55,31 | 6,37 | 5,86 |

***EXEMPLE 5** : 4-{6-[(4-Benzyl-1-pipérazinyl)méthyl]-2,3-diméthoxyphénoxy} butyrate d'éthyle, dichlorhydrate*

Le produit attendu est obtenu selon le procédé décrit dans l'exemple 1 en remplaçant le 7-bromoheptanoate d'éthyle par le 4-bromobutyrate d'éthyle.
*Point de fusion :* 164°C

| Microanalyse élémentaire : | | | |
|---|---|---|---|
| | C% | H% | N% |
| Calculée | 58,98 | 7,23 | 5,29 |
| Trouvée | 59,32 | 7,18 | 5,06 |

***EXEMPLE 6** : Acide 4-{6-[(4-benzyl-1-pipérazinyl)méthyl]-2,3-diméthoxyphénoxy} butyrique, dichlorhydrate*

Le produit attendu est obtenu selon le procédé décrit dans l'exemple 2 à partir du composé décrit dans l'exemple 5.
*Point de fusion* : 200°C

| Microanalyse élémentaire : | | | |
|---|---|---|---|
| | C% | H% | N% |
| Calculée | 57,49 | 6,83 | 5,59 |
| Trouvée | 57,48 | 7,00 | 5,61 |

***EXEMPLE 7 :** 8-{6-[(4-Benzyl-1-pipérazinyl)méthyl]-2,3-diméthoxyphénoxy} octanoate d'éthyle, dichlorhydrate*

Le produit attendu est obtenu selon le procédé décrit dans l'exemple 1 en remplaçant le 7-bromoheptanoate d'éthyle par le 8-bromooctanoate d'éthyle.
*Point de fusion* : 177°C

| Microanalyse élémentaire : | | | |
|---|---|---|---|
| | C% | H% | N% |
| Calculée | 61,53 | 7,92 | 4,78 |
| Trouvée | 61,40 | 7,92 | 4,79 |

***EXEMPLE 8** : Acide 8-{6-[(4-benzyl-1-pipérazinyl)méthyl]-2,3-diméthoxyphénoxy} octanoïque, dichlorhydrate*

Le produit attendu est obtenu selon le procédé décrit dans l'exemple 2 à partir du composé décrit dans l'exemple 7.
*Point de fusion* : 180°C

| *Microanalyse élémentaire :* | | | |
|---|---|---|---|
| | **C%** | **H%** | **N%** |
| *Calculée* | *60,32* | *7,59* | *5,02* |
| *Trouvée* | *60,12* | *7,58* | *5,04* |

***EXEMPLE 9*** ***: 10-{6-[(4-Benzyl-1-pipérazinyl)méthyl]-2,3-diméthoxyphénoxy} décanoate de méthyle, dichlorhydrate***

Le produit attendu est obtenu selon le procédé décrit dans l'exemple 1 en remplaçant le 7-bromoheptanoate d'éthyle par le 10-bromodécanoate de méthyle.
*Point de fusion :* 145°C

| *Microanalyse élémentaire :* | | | |
|---|---|---|---|
| | **C%** | **H%** | **N%** |
| *Calculée* | *62,09* | *8,07* | *4,67* |
| *Trouvée* | *62,15* | *8,17* | *4,67* |

***EXEMPLE 10*** ***: Acide 10-{6-[(4-Benzyl-1-pipérazinyl)méthyl]-2,3-diméthoxyphénoxy} décanoïque, dichlorhydrate***

Le produit attendu est obtenu selon le procédé décrit dans l'exemple 2 à partir du composé décrit dans l'exemple 9.
*Point de fusion :* 156°C

| *Microanalyse élémentaire :* | | | |
|---|---|---|---|
| | **C%** | **H%** | **N%** |
| *Calculée* | *61,53* | *7.92* | *4,78* |
| *Trouvée* | *61,32* | *8,06* | *4,90* |

***EXEMPLE 11*** ***: 11-{6-[(4-Benzyl-1-pipérazinyl)méthyl]-2,3-diméthoxyphénoxy} undécanoate de méthyle, dichlorhydrate***

Le produit attendu est obtenu selon le procédé décrit dans l'exemple 1 en remplaçant le 7-bromoheptanoate d'éthyle par le 11-bromoundécanoate de méthyle.
*Point de fusion :* 177°C

| *Microanalyse élémentaire :* | | | |
|---|---|---|---|
| | **C%** | **H%** | **N%** |
| *Calculée* | *62,63* | *8,21* | *4,57* |
| *Trouvée* | *61,97* | *8,27* | *4,81* |

***EXEMPLE 12*** ***: Acide 11-{6-[(4-benzyl-1-pipérazinyl)méthyl]-2,3-diméthoxyphénoxy} undécanoïque, dichlorhydrate***

Le produit attendu est obtenu selon le procédé décrit dans l'exemple 2 à partir du composé décrit dans l'exemple 11.
*Point de fusion :* 185°C

| *Microanalyse élémentaire :* | | | |
|---|---|---|---|
| | **C%** | **H%** | **N%** |
| *Calculée* | *62,09* | *8,07* | *4,67* |
| *Trouvée* | *61,97* | *8,06* | *4,71* |

***EXEMPLE 13** : 7-{6-{[4-(Pyrimidin-2-yl)-1-pipérazinyl]méthyl}-2,3-diméthoxyphénoxy} heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-{[4-(pyrimidin-2-yl)-1-pipérazinyl]méthyl}-2,3-diméthoxyphénol.
*Point de fusion :* 128°C

| *Microanalyse élémentaire :* | | | |
|---|---|---|---|
| | **C%** | **H%** | **N%** |
| *Calculée* | *55.81* | *7,21* | *10,01* |
| *Trouvée* | *56,11* | *7,40* | *9,96* |

***EXEMPLE 14** : Acide 7-{6-{[4-(pyrimidin-2-yl)-1-pipéraziny])méthyl}-2,3-diméthoxyphénoxy} heptanoïque, dichlorhydrate*

Le produit attendu est obtenu selon le procédé décrit dans l'exemple 2 à partir du composé décrit dans l'exemple 13.
*Point de fusion :* 115°C

| *Microanalyse élémentaire :* | | | |
|---|---|---|---|
| | **C%** | **H%** | **N%** |
| *Calculée* | *54,24* | *6,83* | *10,54* |
| *Trouvée* | *54,33* | *6,90* | *10,63* |

***EXEMPLE 15** : 7-{6-[(Pipérazin-1-yl)méthyl]-2,3-diméthoxyphénoxy} heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-[(4-*tert*-butoxycarbonyl-1-pipérazin)méthyl] -2,3-diméthoxyphénol.

***EXEMPLE 16** : Acide 7-{6-[(pipérazin-1-yl)méthyl]-2,3-diméthoxyphénoxy} heptanoïque, dichlorhydrate*

Le produit attendu est obtenu selon le procédé décrit dans l'exemple 2 à partir du composé décrit dans l'exemple 15.
*Point de fusion :* 160°C

| *Microanalyse élémentaire :* | | | |
|---|---|---|---|
| | **C%** | **H%** | **N%** |
| *Calculée* | *52,98* | *7,56* | *6,18* |
| *Trouvée* | *53,06* | *7,49* | *6,28* |

***EXEMPLE 17** : 7-{6-[(4-Phényl-1-pipérazinyl)méthyl]-2,3-diméthoxyphénoxy} heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-{[(4-phényl)-1-pipérazinyl]méthyl}-2,3-diméthoxyphénol.

***EXEMPLE 18** : Acide 7-{6-[(4-phényl-1-pipérazinyl)méthyl]-2,3-diméthoxyphénoxy} heptanoïque, dichlorhydrate*

Le produit attendu est obtenu selon le procédé décrit dans l'exemple 2 à partir du composé décrit dans l'exemple 17.
*Point de fusion :* 132°C

| *Microanalyse élémentaire :* | | | |
|---|---|---|---|
| | **C%** | **H%** | **N%** |
| *Calculée* | *58,98* | *7,23* | *5,29* |
| *Trouvée* | *58,96* | *7,32* | *5,25* |

***EXEMPLE 19** : 7-{6-[4-Cyclohexyl-1-pipérazinyl)méthyl]-2,3-diméthoxyphénoxy} heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-[(4-cyclohexyl)-1-pipérazinyl)méthyl]-2,3-diméthoxyphénol.

***EXEMPLE 20** : Acide 7-{6-[4-cyclohexyl-1-pipérazinyl)méthyl]-2,3-diméthoxyphénoxy} heptanoïque, dichlorhydrate*

Le produit attendu est obtenu selon le procédé décrit dans l'exemple 2 à partir du composé décrit dans l'exemple 19.
*Point de fusion :* 182°C

| *Microanalyse élémentaire :* | | | |
|---|---|---|---|
| | **C%** | **H%** | **N%** |
| *Calculée* | *58,31* | *8,28* | *5,23* |
| *Trouvée* | *57,92* | *8,41* | *5,24* |

***EXEMPLE 21** : 7-{6-[4-n-Heptyl-1-pipérazinyl)méthyl]-2,3-diméthoxyphénoxy} heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-[(4-n-heptyl)-1-pipérazinyl)méthyl]-2,3-diméthoxyphénol.

***EXEMPLE 22** : Acide 7-{6-[4-n-heptyl-1-pipérazinyl)méthyl]-2,3-diméthoxy phénoxy}heptanoïque, dichlorhydrate*

Le produit attendu est obtenu selon le procédé décrit dans l'exemple 2 à partir du composé décrit dans l'exemple 21.
*Point de fusion :* 152°C

| *Microanalyse élémentaire :* | | | |
|---|---|---|---|
| | **C%** | **H%** | **N%** |
| *Calculée* | *58,79* | *8,77* | *5,08* |
| *Trouvée* | *58,84* | *8,80* | *4,87* |

***EXEMPLE 23** : 7-{6-[4-Cyclopropylméthyl-1-pipérazinyl)méthyl]-2,3-diméthoxy phénoxy}heptanoate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-[(4-cyclopropylméthyl)-1-pipérazinyl)méthyl]-2,3-diméthoxyphénol.

***EXEMPLE 24** : Acide 7-{6-[4-cyclopropylméthyl-1-pipérazinyl)méthyl]-2,3-diméthoxy phénoxy}heptanoïque, dichlorhydrate*

Le produit attendu est obtenu selon le procédé décrit dans l'exemple 2 à partir du composé décrit dans l'exemple 23.
*Point de fusion :* 170°C

| *Microanalyse élémentaire :* | | | |
|---|---|---|---|
| | C% | H% | N% |
| *Calculée* | *58,48* | *8,10* | *5,68* |
| *Trouvée* | *58,20* | *8,11* | *5,53* |

***EXEMPLE 25** : 5-{6-[(4-Benzyl-1-pipérazinyl)méthyl]-2,3-diméthoxy phénoxy}pentanoate d'éthyle, dichlorhydrate*

Le produit attendu est obtenu selon le procédé décrit dans l'exemple 1 en remplaçant le 7-bromoheptanoate d'éthyle par le 5-bromopentanoate d'éthyle.

***EXEMPLE 26** : Acide 5-{6-[(4-benzyl-1-pipérazinyl)méthyl]-2,3-diméthoxy phénoxy}pentanoïque, dichlorhydrate.*

Le produit attendu est obtenu selon le procédé décrit dans l'exemple 2 à partir du composé décrit dans l'exemple 25.
*Point de fusion :* 181°C

| *Microanalyse élémentaire :* | | | |
|---|---|---|---|
| | C% | H% | N% |
| *Calculée* | *58,25* | *7,04* | *5,43* |
| *Trouvée* | *58,28* | *7,07* | *5,47* |

***EXEMPLE 27** : 6-{6-[(4-Benzyl-1-pipérazinyl)méthyl]-2,3-diméthoxyphénoxy} hexanoate d'éthyle, dichlorhydrate*

Le produit attendu est obtenu selon le procédé décrit dans l'exemple 1 en remplaçant le 7-bromoheptanoate d'éthyle par le 6-bromohexanoate d'éthyle.

***EXEMPLE 28** : Acide 6-{6-[(4-benzyl-1-pipérazinyl)méthyl]-2,3-diméthoxy phénoxy}hexanoïque, dichlorhydrate*

Le produit attendu est obtenu selon le procédé décrit dans l'exemple 2 à partir du composé décrit dans l'exemple 27.
*Point de fusion :* 173°C

| *Microanalyse élémentaire :* | | | |
|---|---|---|---|
| | C% | H% | N% |
| *Calculée* | *58,98* | *7,23* | *5,29* |
| *Trouvée* | *59,05* | *7,33* | *5,36* |

***EXEMPLE 29** : 4-{6[(pipérazin-1-yl)méthyl]-2,3-diméthoxyphénoxy}butyrate d'éthyle, dichlorhydrate*

Le produit attendu est obtenu selon le procédé décrit dans l'exemple 1 en remplaçant le 6-[(4-benzyl-1-pipérazinyl)méthyl]-2,3-diméthoxyphénol par le 6-[4-*tert*-butoxycarbonyl-1-pipérazinyl)méthyl]-2,3-diméthoxyphénol et le 7-bromoheptanoate d'éthyle par le 4-bromobutyrate d'éthyle.

***EXEMPLE 30* : Acide 4-{6-[(pipérazin-1-yl)méthyl]-2,3-diméthoxyphénoxy}butyrique, dichlorhydrate**

Le produit attendu est obtenu selon le procédé décrit dans l'exemple 2 à partir du composé décrit dans l'exemple 29.
*Point de fusion* : 141°C

| *Microanalyse élémentaire :* | | | |
|---|---|---|---|
| | **C%** | **H%** | **N%** |
| *Calculée* | *49,64* | *6,86* | *6,81* |
| *Trouvée* | *49,24* | *6,91* | *5,81* |

***EXEMPLE 31* : 4-{6-[(4-Phényl-1-pipérazinyl)méthyl]-2,3-diméthoxyphénoxy butyrate d'éthyle, dichlorhydrate**

Le produit attendu est obtenu selon le procédé décrit dans l'exemple 1 en remplaçant le 6-[(4-benzyl-1-pipérazinyl)méthyl]-2,3-diméthoxyphénol par le 6-[(4-phényl-1-pipérazinyl)méthyl]-2,3-diméthoxyphénol et le 7-bromoheptanoate d'éthyle par le 4-bromobutyrate d'éthyle.

***EXEMPLE 32* : Acide 4-{6-[(4-phényl-1-pipérazinyl)méthyl]-2,3-diméthoxyphénoxy}butyrique, dichlorhydrate**

Le produit attendu est obtenu selon le procédé décrit dans l'exemple 2 à partir du composé décrit dans l'exemple 31.
*Point de fusion* : 129°C

| *Microanalyse élémentaire :* | | | |
|---|---|---|---|
| | **C%** | **H%** | **N%** |
| *Calculée* | *56,68* | *6,62* | *5,75* |
| *Trouvée* | *56,68* | *6,75* | *5,81* |

***EXEMPLE 33* : 7-{6-[(4-(2-Méthoxyphényl)-1-pipérazinyl)méthyl]-2,3-diméthoxyphénoxy}heptanoate d'éthyle, dichlorhydrate**

Le produit attendu est obtenu selon le procédé décrit dans l'exemple 1 en remplaçant le 6-[(4-benzyl-1-pipérazinyl)méthyl]-2,3-diméthoxyphénol par le 6-[4-(2-méthoxyphényl)-1-pipérazinyl)méthyl]-2,3-diméthoxyphénol.

***EXEMPLE 34* : Acide 7-{6-[(4-(2-méthoxyphényl)-1-pipérazinyl)méthyl]-2,3-diméthoxy phénoxy}heptanoïque, dichlorhydrate**

Le produit attendu est obtenu selon le procédé décrit dans l'exemple 2 à partir du composé décrit dans l'exemple 33.
*Point de fusion :* 141°C

| *Microanalyse élémentaire :* | | | |
|---|---|---|---|
| | **C%** | **H%** | **N%** |
| *Calculée* | *57,96* | *7,21* | *5,01* |
| *Trouvée* | *57,70* | *7,22* | *5,02* |

***EXEMPLE 35* : 4-{6-[(4-(2-Méthoxyphényl)-1-pipérazinyl)méthyl]-2,3-diméthoxy phénoxy}butyrate d'éthyle, dichlorhydrate**

Le produit attendu est obtenu selon le procédé décrit dans l'exemple 1 en remplaçant par le 6-[(4-benzyl-1-pipérazinyl)méthyl]-2,3-diméthoxyphénol par le 6-[4-(2-méthoxyphényl)-1-pipérazinyl)méthyl]-2,3-diméthoxyphénol et le 7-bromoheptanoate d'éthyle par le 4-bromobutyrate d'éthyle.

***EXEMPLE 36 : Acide 4-{6-[(4-(2-méthoxyphényl)-1-pipérazinyl)méthyl]-2,3-diméthoxy phénoxy}butyrique, dichlorhydrate***

Le produit attendu est obtenu selon le procédé décrit dans l'exemple 2 à partir du composé décrit dans l'exemple 35.
*Point de fusion :* 153°C

| *Microanalyse élémentaire :* | | | |
|---|---|---|---|
| | **C%** | **H%** | **N%** |
| *Calculée* | *55,71* | *6,62* | *5,41* |
| *Trouvée* | *55,83* | *6,62* | *5,37* |

***EXEMPLE 37 : 4-{6-[(4-(pyrimidin-2-yl))-1-pipérazinyl)méthyl]-2,3-diméthoxy phénoxy}butyrate d'éthyle, dichlorhydrate***

Le produit attendu est obtenu selon le procédé décrit dans l'exemple 1 en remplaçant le 6-[(4-benzyl-1-pipérazinyl)méthyl]-2,3-diméthoxyphénol par le 6-[4-(pyrimidin-2-yl)-1-pipérazinyl)méthyl]-2,3-diméthoxyphénol et le 7-bromoheptanoate d'éthyle par le 4-bromobutyrate d'éthyle.

***EXEMPLE 38 : Acide 4-{6-[(4-(2-pyrimidin-2-yl)-1-pipérazinyl)méthyl]-2,3-diméthoxy phénoxy}butyrique, dichlorhydrate***

Le produit attendu est obtenu selon le procédé décrit dans l'exemple 2 à partir du composé décrit dans l'exemple 37.
*Point de fusion :* 174°C

| *Microanalyse élémentaire :* | | | |
|---|---|---|---|
| | **C%** | **H%** | **N%** |
| *Calculée* | *51,54* | *6,18* | *11,45* |
| *Trouvée* | *51,95* | *5,88* | *11,41* |

***EXEMPLE 39 : 7-{6-[(4-(4-Chlorobenzyl))-1-pipérazinyl)méthyl]-2,3-diméthoxy phénoxy heptanoate d'éthyle, dichlorhydrate***

Le produit attendu est obtenu selon le procédé décrit dans l'exemple 1 en remplaçant le 6-[(4-benzyl-1-pipérazinyl)méthyl]-2,3-diméthoxyphénol par le 6-[4-(4-chlorobenzyl)-1-pipérazinyl)méthyl]-2,3-diméthoxyphénol.

***EXEMPLE 40 : Acide 7-{6-[(4-(4-chlorophényl)-1-pipérazinyl)méthyl]-2,3-diméthoxy phénoxy}heptanoïque, dichlorhydrate***

Le produit attendu est obtenu selon le procédé décrit dans l'exemple 2 à partir du composé décrit dans l'exemple 39.
*Point de fusion :* 187°C

| *Microanalyse élémentaire :* | | | |
|---|---|---|---|
| | **C%** | **H%** | **N%** |
| *Calculée* | *56,11* | *6,80* | *4,85* |
| *Trouvée* | *55,98* | *6,86* | *5,03* |

***EXEMPLE 41: 4-{6-[(4-(4-Chlorophényl))-1-pipérazinyl)méthyl]-2,3-diméthoxy phénoxy}butyrate d'éthyle, dichlorhydrate***

Le produit attendu est obtenu selon le procédé décrit dans l'exemple 1 en remplaçant le 6-[(4-benzyl-1-pipérazinyl)méthyl]-2,3-diméthoxyphénol par le 6-[4-(4-chlorobenzyl)-1-pipérazinyl)méthyl]-2,3-diméthoxyphénol et le

7-bromoheptanoate d'éthyle par le 4-bromobutyrate d'éthyle.

***EXEMPLE 42* : Acide 4-{6-[(4-(4-chlorophényl)-1-pipérazinyl)méthyl]-2,3-diméthoxy phénoxy}butyrique, dichlorhydrate**

Le produit attendu est obtenu selon le procédé décrit dans l'exemple 2 à partir du composé décrit dans l'exemple 41.

***EXEMPLE 43* : 4-{6-[(4-Cyclohexyl-1-pipérazinyl)méthyl]-2,3-diméthoxy phénoxy butyrate d'éthyle, dichlorhydrate**

Le produit attendu est obtenu selon le procédé décrit dans l'exemple 1 en remplaçant le 6-[(4-benzyl-1-pipérazinyl)méthyl]-2,3-diméthoxyphénol par le 6-[(4-cyclohexyl)-1-pipérazinyl)méthyl]-2,3-diméthoxyphénol et le 7-bromoheptanoate d'éthyle par le 4-bromobutyrate d'éthyle.

***EXEMPLE 44* : Acide 4-{6-[(4-cyclohexyl-1-pipérazinyl)méthyl]-2,3-diméthoxy phénoxy}butyrique, dichlorhydrate**

Le produit attendu est obtenu selon le procédé décrit dans l'exemple 2 à partir du composé décrit dans l'exemple 43.
*Point de fusion* : 211°C

| *Microanalyse élémentaire :* | | | |
|---|---|---|---|
| | **C%** | **H%** | **N%** |
| *Calculée* | *55,98* | *7,76* | *5,68* |
| *Trouvée* | *55,74* | *7,70* | *5,70* |

***EXEMPLE 45* : 4-{6-[(4-n-Heptyl-1-pipérazinyl)méthyl]-2,3-diméthoxyphénoxy} butyrate d'éthyle, dichlorhydrate**

Le produit attendu est obtenu selon le procédé décrit à l'exemple 1 en remplaçant le 6-[(4-benzyl-pipérazinyl) méthyl]-2,3-diméthoxyphénol par le 6-[(4-n-heptyl-1-pipérazinyl) méthyl]-2,3-diméthoxyphénol et le 7-bromoheptanoate d'éthyle par le 4-bromobutyrate d'éthyle.

***EXEMPLE 46* : Acide 4-{6-[(4-n-heptyl-1-pipérazinyl)méthyl]-2,3-diméthoxy phénoxy}butyrique, dichlorhydrate**

Le produit attendu est obtenu selon le procédé décrit à l'exemple 2 à partir du composé décrit à l'exemple 45.

***EXEMPLE 47* : 4-{6-[(4-Cyclopropylméthyl-1-pipérazinyl)méthyl]-2,3-diméthoxyphénoxy}butyrate d'éthyle, dichlorhydrate**

Le produit attendu est obtenu selon le procédé décrit à l'exemple 1 en remplaçant le 6-[(4-benzyl-pipérazinyl) méthyl]-2,3-diméthoxyphénol par le 6-[(4-cyclopropylméthyl-1-pipérazinyl)méthyl]-2,3-diméthoxyphénol et le 7-bromoheptanoate d'éthyle par le 4-bromobutyrate d'éthyle.

***EXEMPLE 48* : Acide 4-{6-[(4-cyclopropylméthyl-1-pipérazinyl)méthyl]-2,3-diméthoxy phénoxy}butyrique, dichlorhydrate**

Le produit attendu est obtenu selon le procédé décrit à l'exemple 2 à partir du composé décrit à l'exemple 47.

***EXEMPLE 49* : 3-{6-[(4-Benzyl-1-pipérazinyl)méthyl]-2,3-diméthoxyphénoxy} propionate d'éthyle, dichlorhydrate**

Le produit attendu est obtenu selon le procédé décrit à l'exemple 1 en remplaçant le 7-bromoheptanoate d'éthyle par le 3-bromopropionate d'éthyle.

***EXEMPLE 50* : Acide 3-{6-[(4-benzyl-1-pipérazinyl)méthyl]-2,3-diméthoxy phénoxy}propionique, dichlorhydrate**

Le produit attendu est obtenu selon le procédé décrit à l'exemple 2 à partir du composé décrit à l'exemple 49.

***EXEMPLE 51* : 7-{6-[(4-(2,6-dipyrolidinyl-pyrimidin-4-yl)-1-pipérazinyl)méthyl]-2,3-diméthoxyphénoxy}heptanoate d'éthyle, tétrachlorhydrate**

Le produit attendu est obtenu selon le procédé décrit à l'exemple 1 en remplaçant le 6-[(4-benzyl-1-pipérazinyl)méthyl]-2,3-diméthoxyphénol par le 6-[(4-2,6-dipyrolidinylpyrimidin-4-yl)-1-pipérazinyl)méthyl]-2,3-diméthoxyphénol.

***EXEMPLE 52* : Acide 7-{6-[(4-(2,6-dipyrolidinyl-pyrimidin-4-yl)-1-pipérazinyl)méthyl]-2,3-diméthoxyphénoxy} heptanoïque, tétrachlorhydrate**

Le produit attendu est obtenu selon le procédé décrit à l'exemple 2 à partir du composé décrit à l'exemple 51.

***EXEMPLE 53* : 4-{6-[(4-(2,6-dipyrolidinyl-pyrimidin-4-yl)-1-pipérazinyl)méthyl]-2,3-diméthoxyphénoxy}butyrate d'éthyle, tétrachlorhydrate**

Le produit attendu est obtenu selon le procédé décrit à l'exemple 1 en remplaçant le 6-[(4-benzyl-1-pipérazinyl)méthyl]-2,3-diméthoxyphénol par le 6-[(4-(2,6-dipyrolidinylpyrimidin-4-yl)-1-pipérazinyl)méthyl]-2,3-diméthoxyphénol et le 7-bromoheptanoate d'éthyle par le 4-bromobutyrate d'éthyle.

***EXEMPLE 54*: Acide 4-{6-[(4-(2,6-dipyrolidinyl-pyrimidin-4-yl)-1-pipérazinyl)méthyl]-2,3-diméthoxyphénoxy} butyrique, tétrachlorhydrate**

Le produit attendu est obtenu selon le procédé décrit à l'exemple 2 à partir du composé décrit à l'exemple 53.

***EXEMPLE 55* : 7-{6-[(4-n-(6-Ethoxycarbonyl)hexyl-1-pipérazinyl)méthyl]-2,3-diméthoxyphénoxy}heptanoate d'éthyle, dichlorhydrate**

Le produit attendu est obtenu selon le procédé décrit à l'exemple 1 en remplaçant le 6-[(4-benzyl-pipérazinyl) méthyl]-2,3-diméthoxyphénol par le 6-[(4-n-(6-éthoxycarbonyl)hexyl-1-pipérazinyl)méthyl]-2,3-diméthoxyphénol.

***EXEMPLE 56* : Acide 7-{6-[(4-n-(6-carboxy)hexyl-1-pipérazinyl)méthyl]-2,3-diméthoxyphénoxy}heptanoïque, dichlorhydrate**

Le produit attendu est obtenu selon le procédé décrit à l'exemple 2 à partir du composé décrit à l'exemple 55.

***EXEMPLE 57*: 4-{6-[(4-(3-Ethoxycarbonyl)propyl-1-pipérazinyl)méthyl]-2,3-diméthoxyphénoxy}butyrate d'éthyle, dichlorhydrate**

Le produit attendu est obtenu selon le procédé décrit à l'exemple 1 en remplaçant le 6-[(4-benzyl-pipérazinyl) méthyl]-2,3-diméthoxyphénol par le 6-[(4-(3-éthoxycarbonyl)propyl-1-pipérazinyl)méthyl]-2,3-diméthoxyphénol et le 7-bromoheptanoate d'éthyle par le 4-bromobutyrate d'éthyle.

***EXEMPLE 58* : Acide 4-{6-[(4-(3-carboxy)propyl-1-pipérazinyl)méthyl]-2,3-diméthoxy phénoxy}butyrique, dichlorhydrate**

Le produit attendu est obtenu selon le procédé décrit à l'exemple 2 à partir du composé décrit à l'exemple 57.

***EXEMPLE 59* : 7-{6-[(4-(3-Ethoxycarbonyl)propyl-1-pipérazinyl)méthyl]-2,3-diméthoxyphénoxy}heptanoate d'éthyle, dichlorhydrate**

Le produit attendu est obtenu selon le procédé décrit à l'exemple 1 en remplaçant le 6-[(4-benzyl-pipérazinyl) méthyl]-2,3-diméthoxyphénol par le 6-[(4-(3-éthoxycarbonyl)propyl-1-pipérazinyl)méthyl]-2,3-diméthoxyphénol.

***EXEMPLE 60*** ***: Acide 7-{6-[(4-(3-carboxy)propyl-1-pipérazinyl)méthyl]-2,3-diméthoxyphénoxy}heptanoïque, dichlorhydrate***

Le produit attendu est obtenu selon le procédé décrit à l'exemple 2 à partir du composé décrit à l'exemple 59.

***EXEMPLE 61*** ***: 4-{6-[(4-n-(6-Ethoxycarbonyl)hexyl-1-pipérazinyl)méthyl]-2,3-diméthoxyphénoxy}butyrate d'éthyle, dichlorhydrate***

Le produit attendu est obtenu selon le procédé décrit à l'exemple 1 en remplaçant le 6-[(4-benzyl-pipérazinyl) méthyl]-2,3-diméthoxyphénol par le 6-[(4-n-(6-éthoxycarbonyl)hexyll-pipérazinyl)méthyl]-2,3-diméthoxyphénol et le 7-bromoheptanoate d'éthyle par le 4-bromobutyrate d'éthyle.

***EXEMPLE 62*** ***: Acide 4-{6-[(4-n-(6-carboxy)hexyl-1-pipérazinyl)méthyl]-2,3-diméthoxyphénoxy}butyrique, dichlorhydrate***

Le produit attendu est obtenu selon le procédé décrit à l'exemple 2 à partir du composé décrit à l'exemple 61.

***EXEMPLE 63*** ***: 7-{6-[(4-Benzyl-1-pipérazinyl)méthyl]-2,3-diméthoxyphénoxy}-N,N-diéthylheptanamide, dichlorhydrate***

4,95 g du composé décrit dans l'exemple 2 sont mis en solution dans 100 ml de dichlorométhane. On ajoute 2,17 g de dicyclohexylcarbodiimide puis 2,1 ml de N,N-diéthyl amine et 1,28 g de N,N-diméthylaminopyridine. Après 24 h d'agitation, la solution est filtrée, puis concentrée à sec et le résidu brut obtenu est purifié par chromatographie sur colonne de silice en utilisant un gradiant d'acétate d'éthyle/méthanole (100/0 à 80/20) pour conduire au produit attendu sous forme de base qui est transformé en dichlorhydrate correspondant.

***EXEMPLE 64*** ***: 4-{6-[(4-Benzyl-1-pipérazinyl)méthyl]-2,3-diméthoxyphénoxy}-N,N-diéthylbutanamide, dichlorhydrate***

Le produit attendu est obtenu selon le procédé décrit à l'exemple 63 à partir du composé décrit dans l'exemple 6.
*Point de fusion :* 198°C

| *Microanalyse élémentaire :* | | | |
|---|---|---|---|
| | **C%** | **H%** | **N%** |
| *Calculée* | *60,41* | *7,79* | *7,55* |
| *Trouvée* | *60,17* | *7,79* | *7,50* |

***EXEMPLE 65*** ***: 3-{6-[(4-Benzyl-1-pipérazinyl)méthyl]-2,3-diméthoxyphénoxy} propionamide, dichlorhydrate***

Le produit attendu est obtenu à partir du composé décrit dans l'exemple 50 qui est mis en réaction en milieu ammoniacal.

## ETUDE PHARMACOLOGIQUE DES DERIVES DE L'INVENTION

### A : Etude de l'activité anti-ischémique des composés de l'invention

Le modèle utilisé est un modèle d'ischémie cérébrale focale transitoire par occlusion intraluminal qui mime l'accident vasculaire cérébral embolique chez l'homme. La localisation de l'infarctus est corticale et sous-corticale. (R. Bordet et coll., *J Cereb Blood Flow Metab. 20*, 2000, 1190-1196 et 1999, *19*, 1309-1345).
Le modèle consiste en une occlusion unilatérale de l'artère moyenne carotidienne pendant 1 heure suivi d'une reperfusion pendant 23 heures chez le rat male Wistar adulte. Le but de cette expérience est d'étudier l'effet anti-ischémique des composés de l'invention.

Les animaux sont anesthésiés pour la chirurgie avec 300 mg/kg d'hydrate de chloral. Pendant la durée de la chirurgie, la température est contrôlée et maintenue à 37 °C à l'aide d'une couverture chauffante thermostatée.

La procédure principale consiste à stopper la circulation sanguine vers l'artère cérébrale moyenne (ACM)

droite par une occlusion intraluminale de l'ostium. Après 60 minutes l'occlusion est levée et les animaux sont replacés dans leur cage. Après 24 h les animaux sont sacrifiés sous anesthésie. Les cerveaux sont rapidement prélevés, congelés et 12 coupes d'une épaisseur de 50 μm et à une distance de 1 μm les unes des autres sont obtenues sur un cryostat selon la topographie stéréotaxique. Les coupes sont colorées avec du violet Crésyl rapide. Les régions non-colorées des coupes cérébrales sont définies comme les zones infarcies.
Les zones infarcies non corrigées et les zones hémisphériques totales sont calculées pour chaque coupe séparément à l'aide d'un programme d'analyse d'imagerie. Le volume d'infarctus total corrigé est calculé afin de compenser l'effet oedème cérébral.

Le produit est administré à 1 mg/kg par voie intraveineuse 3 minutes avant l'occlusion.

Dans ce modèle, les composés de l'invention et notamment le composé de l'exemple 2 ont réduit significativement la taille de l'infarctus du cortex et du striatum, indiquant un effet neuroprotecteur.

**B : Hypoxie des astrocytes en culture**

Protocole expérimental :

Les cultures d'astrocytes sont obtenues à partir de rats nouveau-nés d'après la technique décrite par Booher et Sensenbrenner (Neurobiology., 1972, 2, 97-105).

Elles sont utilisées trois à six semaines après la mise en culture selon un modèle d'hypoxie décrite par Plateel et coll. (J. Neurochem. 1995, 65, 2138-2145). Le milieu est changé 24 heures avant la mise en hypoxie, afin de reconstituer les réserves ATP. Après addition de milieu de culture dégazé en O2 et contenant une concentration donnée de molécules à tester, l'hypoxie est réalisée dans un caisson anaérobie pour une durée de 24 heures. La concentration en ATP est déterminée en luminescence et exprimée en picomoles d'ATP/milligramme de protéine.

Dans ce modèle, pour des degrés d'hypoxie allant de 27% à 79%, le composé de l'exemple 2 permet une récupération allant de 35 à 87% de l'ATP alors que la trimétazidine n'a aucun effet sur ce paramètre. L'effet maximal est observé à 1 μM.

**C : Neuroprotection dans le cortex et la substance blanche par les composés de l'invention**

Le modèle utilisé est un modèle murin de neurodégénérescence touchant à la fois les neurones corticaux et la substance blanche sous-jacente (S.L. Tahraoui et coll., Brain Pathol., 2001, 11, 56-71). Ce modèle repose sur l'injection intracérébrale de S-bromo-willardiine, un agoniste glutamatergique agissant sur les récepteurs AMPA-kaïnate, ou d'iboténate, un agoniste des récepteurs de type NMDA, chez le souriceau nouveau-né.

Toutes les injections sont effectuées le matin entre 10h et 13h. Les animaux sont préalablement anesthésiés et maintenus sous une lampe chauffante le temps des manipulations. Chaque groupe expérimental est composé de 5 à 12 souriceaux Swiss des deux sexes, issus d'au moins 2 portées différentes. Au 5$^{ème}$ jour de vie (P5), 10 μg d'iboténate ou 15 μg de S-bromo-willardiine sont injectés en intracérébral au moyen d'une aiguille de calibre 25G montée sur une seringue de 50μl, fixée à un dispositif calibré délivrant à chaque pression un bolus de 1 μl. L'aiguille est enfoncée 2 mm en dessous du plan cutané, dans la région fronto-pariétale de l'hémisphère droit (2mm en avant de la suture lambdoïde et 2mm en dehors de la ligne médiane). Deux bolus de 1μl (1μl dans la substance blanche périventriculaire et 1μl dans le cortex) sont délivrés. Un délai de 30 secondes, où l'aiguille reste en place, sépare chaque bolus afin de permettre la diffusion du produit. Immédiatement après cette agression excitotoxique, le composé à tester (3, 10 ou 30 mg/kg) ou le véhicule sont administrés par voie intrapéritonéale (volume injecté = 5 μl). Les animaux sont sacrifiés par décapitation 5 jours plus tard (P10) et les cerveaux sont immédiatement prélevés et fixés dans du formol avant d'être inclus en paraffine. Des coupes coronales sériées de 15 μm sont réalisées sur l'ensemble des cerveaux et colorées au crésyl-violet, permettant de déterminer, de manière précise et reproductible, l'axe antério-postérieur des lésions histologiques tant au niveau cortical que de la substance blanche. Les tailles des lésions histologiques sont déterminées par deux observateurs indépendants n'ayant pas connaissance des groupes expérimentaux. Les résultats sont exprimés sous forme de moyennes.
Dans ce modèle le composé de l'exemple 2 notamment a réduit la taille des lésions dans le substance blanche et le cortex ce qui indique un effet neuroprotecteur important. La trimétazidine (TMZ) testée à titre comparatif présente un très faible effet neuroprotecteur .

| Produit, 10mg/kg i.p. | Lésions corticales | | | Lésions substance blanche | | |
|---|---|---|---|---|---|---|
| | contrôle | TMZ | composé de l'exemple 2 | contrôle | TMZ | composé de l'exemple 2 |
| Nombre d'animaux | 19 | 7 | 6 | 18 | 7 | 6 |
| lésion, µm | 850,5 | 800 | 200 | 480 | 320 | 146,7 |

**D : Stabilité métabolique**

Le modèle utilisé pour estimer la stabilité métabolique a été développé et validé dans des microsomes de rat et d'homme isolés (M. Bertrand et coll., European Journal of Pharmaceutical Sciences, 2000, Vol 11, Suppl. 2, S61-S72).

La prédiction de la biodisponibilité métabolique (MF%) est basée sur la mesure in vitro de la stabilité métabolique avec des microsomes hépatiques en supposant une absorption totale. Brièvement, le produit est incubé à $10^{-7}$M en présence de microsomes hépatiques de rat et d'homme (0.33 mg protéine/ml) pendant 0, 5, 15, 30 et 60 minutes. A chaque temps, la quantité de produit inchangé est mesuré par LC-MS-MS. La clairance intrinsèque in vitro (vitroClint) correspond à la pente (après linéarisation LN) de la concentration restante en produit inchangé versus le temps d'incubation. Cette clairance intrinsèque in vitro est alors transposée à l'organisme entier en clairance intrinsèque in vivo (vivoClint) en utilisant les facteurs 0.045 mg prot/kg de foie et un poids de foie de 11 g pour le rat et de 1.2 kg pour l'homme. La transformation de la clairance intrinsèque in vivo en clairance hépatique (HepCl) est obtenue à l'aide du modèle de l'équilibre veineux (HepCl=vivoClint*HBF/(vivoClint + HBF) où HBF (débit sanguin hépatique) correspond à 22 ml/min pour le rat et à 1500 ml/min pour l'homme. La MF% est alors déduite du coefficient d'extraction hépatique avec la formule suivante :

MF%=1-HepCl/HBF.

Cette méthode donne une bonne corrélation entre les données in vitro et in vivo.

Dans ce modèle les composés de l'invention ont montré une très bonne stabilité métabolique.

**E- Effet anti-apoptotique**

Une étude in vitro recherchant un effet anti-apoptotique au sein de cultures primaires de cardiomyocytes a été réalisée en induisant l'apoptose par l'addition d'eau oxygénée ($H_2O_2$). Les radicaux libres produits induisent l'apoptose. Celle-ci se rapproche de ce que l'on observe lors d'une ischémie-reperfusion (R. Von Harsdorf, Circulation 99, 2934-2941).

Les cardiomyocytes sont isolés à partir de coeurs de rats nouveau-nés et ensemencés à une densité de 35 000 cellules/$cm^2$. Le processus d'apoptose est induit par l'addition de $H_2O_2$ (0,075 mmol/L) pendant 30 minutes. Puis les cardiomyocytes sont replacés 24h dans le milieu de culture. Les cellules apoptotiques sont visualisées par différentes techniques, par exemple par la fragmentation de l'ADN génomique qui survient lors du processus de l'apoptose. Elle est mise en évidence par les deux techniques suivantes (référence ci-dessus) :

1 Le test TUNEL permet de marquer les noyaux cellulaires présentant une fragmentation de leur ADN.
2 La fragmentation de l'ADN est mise en évidence par la technique de "DNA laddering". Les fragments caractéristiques de 180 bp de l'ADN génomique sont détectés par électrophorèse sur gel d'agarose.

La migration de la phosphatidylsérine (PS) de l'intérieur de la membrane du noyau cellulaire vers l'extérieur (externalisation) est un indice précoce de l'apoptose. Celui-ci peut être visualisé par un marquage fluorescent à l'annexine-V-FITC (Fluorescein Isothiocyanate) et quantifié par rapport au nombre total de cellules.
Dans ce modèle in vitro, le composé de l'exemple 2 protège les cardiomyocytes de l'apoptose. L'effet se traduit à la fois par une diminution du pourcentage de cellules en apoptose et par une augmentation de la survie cellulaire. En conséquence, le produit est cardioprotecteur.

**F- Neuroprotection dans les régions CA1 et CA3 de l'hippocampe**

L'acide kaïnique est une des plus puissantes excitotoxines exogènes qui soient, ce dont témoigne son aptitude

à induire des troubles épilepiformes aigus ou subaigus. Les modifications histopathologiques observées liées à une administration de l'acide kaïnique se différencient de l'ischémie cérébrale par des lésions qui se manifestent d'abord dans les régions CA1 à CA3 de l'hippocampe (C. Montecot et coll., Neuroscience 84, 791-800). Le but est d'étudier l'effet protecteur vis-à-vis des lésions induites essentiellement dans la région CA3 de l'hippocampe alors que la circulation sanguine n'est pas réduite.

L'acide kaïnique est administré par voie intrapéritonéale à la dose de 12 mg/kg chez le rat vigile. L'animal est ensuite observé pendant une période d'environ 2 heures pendant laquelle se manifestent, après une période de latence, des convulsions épilepiformes. Au bout des 2 heures, les convulsions sont arrêtées par l'administration de 10 mg/kg de diazépam. Les animaux sont sacrifiés 7 jours plus tard, les cerveaux isolés et 8 coupes transversales de 15 µm d'épaisseur, espacées de 150 µm, sont réalisées. Celles-ci sont analysées en microscopie de contraste par deux observateurs indépendants n'ayant pas connaissance des groupes expérimentaux. Un score de 0 à 3 est attribué selon la sévérité des lésions neuronales. Les scores de chaque hémisphère sont additionnés afin d'obtenir un score lésionnel.

Le produit est administré à raison de 20 mg/kg par voie intrapéritonéale, 3 minutes avant l'administration de l'acide kaïnique.

Dans ce modèle, une injection unique du composé de l'exemple 2 protège les neurones des régions CA1 et CA3 de l'hippocampe des lésions induites par l'acide kaïnique et montre donc un fort effet neuroprotecteur. ***<u>Composition pharmaceutique</u>:***

| Formule de préparation pour 1000 comprimés dosés à 10 mg | |
|---|---|
| Composé de l'exemple 2 | 10 g |
| Hydroxypropylcellulose | 2g |
| Amidon de blé | 10 g |
| Lactose | 100g |
| Stéarate de magnésium | 3g |
| Talc | 3g |

## Revendications

1. Composés de formule (I) :

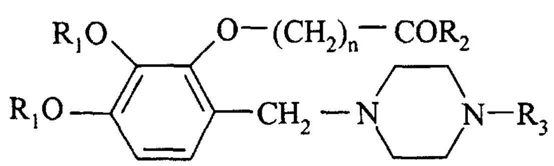

(I)

dans laquelle :

$R_1$ représente un groupement alkyle ($C_1$-$C_6$) linéaire ou ramifié,
$R_2$ représente un groupement hydroxy, alkoxy ($C_1$-$C_6$) linéaire ou ramifié, ou amino (éventuellement substitué par un ou deux groupements alkyle ($C_1$-$C_6$) linéaire ou ramifié),
n représente un nombre entier compris entre 1 et 12 inclusivement.
$R_3$ représente :

- un atome d'hydrogène,
- un groupement alkyle ($C_1$-$C_{12}$) linéaire ou ramifié éventuellement substitué par un groupement phényle (lui-même éventuellement substitué par un ou plusieurs, identiques ou différents, atomes d'halogène ou groupements alkyle ($C_1$-$C_6$) linéaire ou ramifié, alkoxy ($C_1$-$C_6$) linéaire ou ramifié, hydroxy ou trihalogènométhyle), cycloalkyle ($C_3$-$C_7$), carboxy ou alkoxy($C_1$-$C_6$)carbonyle,
- un groupement cycloalkyle ($C_3$-$C_7$),
- un groupement pyrimidinyle éventuellement substitué par un ou deux groupements pyrolidinyle,
- ou, un groupement phényle éventuellement substitué par un ou plusieurs, identiques ou différents, atomes d'halogène ou groupements alkyle ($C_1$-$C_6$) linéaire ou ramifié, alkoxy ($C_1$-$C_6$) linéaire ou ramifié, hydroxy ou trihalogènométhyle,

leurs isomères optiques ainsi que leurs sels d'addition à un acide ou à une base pharmaceutiquement acceptable.

**2.** Composés de formule (I) selon la revendication 1 tel que $R_1$ représente un groupe méthyle.

**3.** Composés de formule (I) selon la revendication 1 tel que $R_3$ un groupement alkyle substitué par un groupement phényle préférentiellement un groupement benzyle, un groupement pyrimidinyle, un groupement phényle avantageusement substitué par un groupement méthoxy ou un atome d'hydrogène.

**4.** Composés de formule (I) selon la revendication 1 tel que n est un nombre entier compris entre 1 et 7.

**5.** Composés de formule (I) selon la revendication 4 tel que n est égal à 6.

**6.** Composés de formule (I) selon la revendication 1 tel que $R_2$ représente un groupement hydroxy.

**7.** Composé de formule (I) selon la revendication 1 qui est l'acide 7-{6-[(4-benzyl-1-pipérazinyl)méthyl]-2,3-diméthoxyphénoxy} heptanoïque, ainsi que ses sels d'addition.

**8.** Composés de formule (I) selon la revendication 1 qui sont l'acide 7-{6-{[4-(pyrimidin-2-yl)-1-pipéraziny])méthyl} - 2,3-diméthoxyphénoxy} heptanoïque, l'acide 4-{6-[(pipérazin-1-yl)méthyl]-2,3-diméthoxyphénoxy}butyrique, l'acide 7-{6-[(4-(2-méthoxyphényl)-1-pipérazinyl)méthyl]-2,3-diméthoxyphénoxy} heptanoïque, l'acide 4-{6-[(4-(2-méthoxyphényl)-1-pipérazinyl)méthyl]-2,3-diméthoxyphénoxy}butyrique, ainsi que leurs sels d'addition

**9.** Procédé de préparation des composés de formule (I) selon la revendication 1 **caractérisé en ce que** l'on utilise comme produit de départ un composé de formule (II):

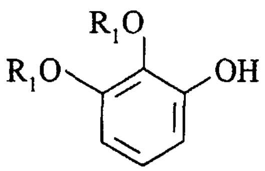

(II)

dans laquelle $R_1$ a la même signification que dans la formule (I), que l'on fait réagir avec une pipérazine substituée de formule (III) :

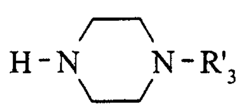

(III)

dans laquelle $R'_3$ a la même signification que $R_3$ à l'exception d'un groupement alkyle substitué par un groupement carboxy, en présence de formaldéhyde, pour conduire au composé de formule (IV) :

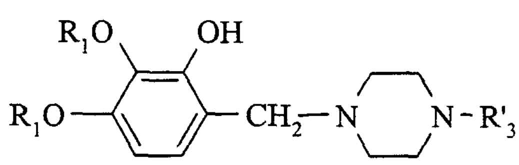

(IV)

dans laquelle $R_1$ et $R'_3$ ont la même signification que précédemment, que l'on fait réagir avec un composé de formule (V) :

$$X\text{-}(CH_2)_n\text{-}COR'_2 \quad (V)$$

dans laquelle X représente un atome d'halogène et $R'_2$ représente un groupement alkoxy ($C_1$-$C_6$) linéaire ou ramifié, pour conduire au composé de formule (I/a) cas particulier des composés de formule (I) :

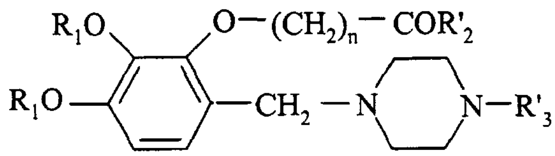
(I/a)

dans laquelle $R_1$, $R'_2$, $R'_3$ et n ont la même signification que précédemment, composé de formule (I/a) que l'on transforme si on le souhaite

- soit en mono ou diacide correspondant de formule (I/b), cas particulier des composés de formule (I) :

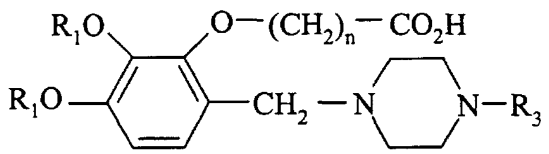
(I/b)

- soit en amide correspondant de formule (I/c):

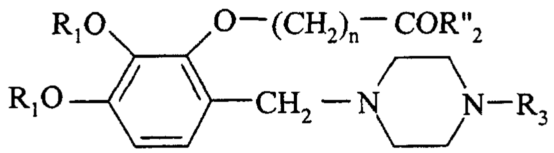
(I/c)

dans laquelle $R_1$, $R_3$ et n ont la même signification que dans la formule (I) et $R''_2$ représente un groupement amino éventuellement substitué par un ou deux groupements alkyle ($C_1$-$C_6$) linéaire ou ramifié,
composés de formule (I/a) (I/b) ou (I/c),
que l'on purifie, le cas échéant, selon une technique classique de purification, dont on sépare éventuellement les isomères, et que l'on transforme, si on le souhaite, en sel d'addition à un acide ou une base pharmaceutiquement acceptable.

**10.** Compositions pharmaceutiques contenant comme principe actif un composé selon l'une quelconque des revendications 1 à 8 en combinaison avec un ou plusieurs excipients ou véhicules inertes, non toxiques, pharmaceutiquement acceptables.

**11.** Compositions pharmaceutiques selon la revendication 10 contenant un principe actif selon l'une quelconque des revendications 1 à 8 utiles pour la prévention ou le traitement de l'ischémie cellulaire aigue et chronique, des accidents ischémiques aigus ou chroniques, cérébraux, cardiaques ou périphériques suivis ou non de reperfusion, avant toute intervention chirurgicale nécessitant un arrêt transitoire de la circulation sanguine, pour le traitement des maladies neurodégénératives chroniques, ainsi que pour l'amélioration de la conservation d'organes destinés à être transplantés et la reprise fonctionnelle des greffons après reperfusion.

## Patentansprüche

**1.** Verbindungen der Formel (I):

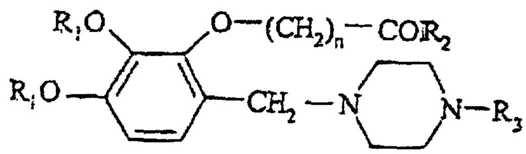

(I)

in der:

$R_1$ eine geradkettige oder verzweigte ($C_1$-$C_6$)-Alkylgruppe bedeutet,
$R_2$ eine Hydroxygruppe, geradkettige oder verzweigte ($C_1$-$C_6$)-Alkoxygruppe oder Aminogruppe (die gegebenenfalls durch eine oder zwei geradkettige oder verzweigte ($C_1$-$C_6$)-Alkylgruppen substituiert ist) bedeutet,
n eine ganze Zahl zwischen 1 und 12 einschließlich bedeutet,
$R_3$:

- ein Wasserstoffatom,
- eine geradkettige oder verzweigte ($C_1$-$C_{12}$)-Alkylgruppe, die gegebenenfalls durch eine Phenylgruppe (die ihrerseits gegebenenfalls durch ein oder mehrere, gleichartige oder verschiedenartige Halogenatome oder geradkettige oder verzweigte ($C_1$-$C_6$)-Alkyl-, geradkettige oder verzweigte ($C_1$-$C_6$)-Alkoxy-, Hydroxy- oder Trihalogenmethylgruppen substituiert ist), ($C_3$-$C_7$)-Cycloalkylgruppe, Carboxygruppe oder ($C_1$-$C_6$) -Alkoxycarbonylgruppe substituiert ist,
- eine ($C_3$-$C_7$)-Cycloalkylgruppe,
- eine Pyrimidinylgruppe, die gegebenenfalls durch eine oder zwei Pyrrolidinylgruppen substituiert ist,
- oder eine Phenylgruppe, die gegebenenfalls durch ein oder mehrere, gleichartige oder verschiedenartige Halogenatome oder geradkettige oder verzweigte ($C_1$-$C_6$)-Alkyl-, geradkettige oder verzweigte ($C_1$-$C_6$) -Alkoxy-, Hydroxy- oder Trihalogenmethylgruppen substituiert ist, bedeutet,

der optische Isomere sowie deren Additionssalze mit einer pharmazeutisch annehmbaren Säure oder Base.

**2.** Verbindungen der Formel (I) nach Anspruch 1, worin $R_1$ eine Methylgruppe bedeutet.

**3.** Verbindungen der Formel (I) nach Anspruch 1, worin $R_3$ eine durch eine Phenylgruppe substituierte Alkylgruppe, vorzugsweise eine Benzylgruppe, eine Pyrimidinylgruppe, eine vorzugsweise durch eine Methoxygruppe substituierte Phenylgruppe oder ein Wasserstoffatom bedeutet.

**4.** Verbindungen der Formel (I) nach Anspruch 1, worin n eine ganze Zahl zwischen 1 und 7 bedeutet.

**5.** Verbindungen der Formel (I) nach Anspruch 4, worin n den Wert 6 besitzt.

**6.** Verbindungen der Formel (I) nach Anspruch 1, worin $R_2$ eine Hydroxygruppe bedeutet.

**7.** Verbindung der Formel (I) nach Anspruch 1, nämlich 7-{6-[(4-Benzyl-1-piperazinyl)-methyl]-2,3-dimethoxyphenoxy}-heptansäure sowie deren Additionssalze.

**8.** Verbindungen der Formel (I) nach Anspruch 1, nämlich 7-{6-{[4-(Pyrimidin-2-yl)-1-piperazinyl])-methyl}-2,3-dimethoxyphenoxy}-heptansäure, 4-{6-[(Piperazin-1-yl)-methyl]-2,3-dimethoxyphenoxy}-buttersäure, 7-{6-[(4-(2-Methoxyphenyl)-1-piperazinyl)-methyl]-2,3-dimethoxyphenoxy}-heptansäure, 4-{6-[(4-(2-Methoxyphenyl)-1-piperazinyl)-methyl]-2,3-dimethoxyphenoxy}-buttersäure sowie deren Additionssalze.

**9.** Verfahren zur Herstellung der Verbindungen der Formel (I) nach Anspruch 1. **dadurch gekennzeichnet, daß** man als Ausgangsprodukt eine Verbindung der Formel (II) verwendet:

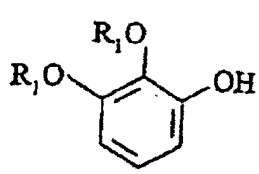

(II)

in der $R_1$ die bezüglich der Formel (I) angegebenen Bedeutungen besitzt, welche man mit einem substituierten Piperazin der Formel (III):

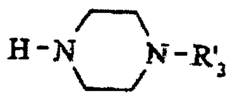

(III)

in der $R'_3$ die gleiche Bedeutung besitzt wie $R_3$ mit Ausnahme der durch eine Carboxygruppe substituierten Alkylgruppe, in Gegenwart von Formaldehyd umsetzt, zur Bildung der Verbindung der Formel (IV):

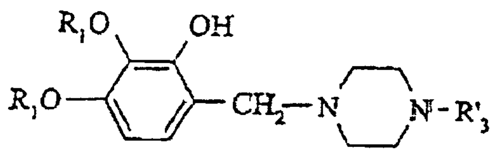

(IV)

in der $R_1$ und $R'_3$ die oben angegebenen Bedeutungen besitzen, welche man mit einer Verbindung der Formel (V) umsetzt:

$$X - (CH_2)_n - COR'_2 \qquad (V)$$

in der X ein Halogenatom und $R'_2$ eine geradkettige oder verzweigte ($C_1$-$C_6$)-Alkoxygruppe bedeuten, zur Bildung der Verbindung der Formel (I/a), einem Sonderfall der Verbindungen der Formel (I):

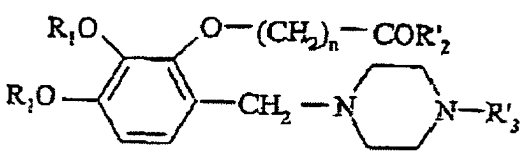

(I/a)

in der $R_1$, $R'_2$, $R'_3$ und n die oben angegebenen Bedeutungen besitzen, welche Verbindung der Formel (I/a) man gewünschtenfalls:

- entweder in die entsprechende Mono- oder Di-säure der Formel (I/b) umwandelt, einem Sonderfall der Verbindungen der Formel (I):

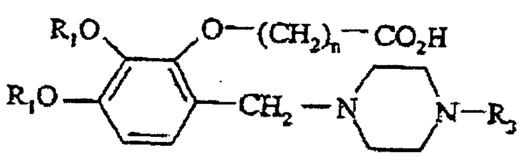

(I/b)

- oder in das entsprechende Amid der Formel (I/c):

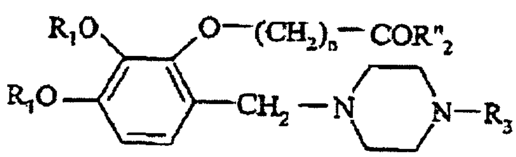

(I/c)

in denen $R_1$, $R_3$ und n die bezüglich der Formel (I) angegebenen Bedeutungen besitzen und $R''_2$ eine gegebenen-

falls durch eine oder zwei geradkettige oder verzweigte ($C_1$-$C_6$)-Alkylgruppen substituierte Aminogruppe bedeutet, welche Verbindungen der Formeln (I/a), (I/b) oder (I/c)
man gegebenenfalls mit Hilfe einer klassischen Reinigungsmethode reinigt, gegebenenfalls in ihre Isomeren auftrennt und gewünschtenfalls in ihre Additionssalze mit einer pharmazeutisch annehmbaren Säure oder Base umwandelt.

**10.** Pharmazeutische Zubereitungen enthaltend als Wirkstoff eine Verbindung nach einem der Ansprüche 1 bis 8 in Kombination mit einem oder mehreren inerten, nichttoxischen, pharmazeutisch annehmbaren Trägermaterialien oder Bindemitteln.

**11.** Pharmazeutische Zubereitungen nach Anspruch 10, enthaltend einen Wirkstoff nach irgendeinem der Ansprüche 1 bis 8 nützlich für die Vorbeugung oder die Behandlung von akuter und chronischer Zellischämie, akuten oder chronischen, das Gehirn, das Herz oder periphere Bereiche betreffenden ischämischen Vorfällen, die gegebenenfalls von einer Reperfusion gefolgt sind, vor einem chirurgischen Eingriff, der eine vorübergehende Unterbrechung der Blutzirkulation erforderlich macht, zur Behandlung von chronischen neurodegenerativen Erkrankungen sowie zur Verbesserung der Aufbewahrung von Organen, die für die Transplantation bestimmt sind, und für die Funktionsaufnahme von transplantierten Organen nach der Reperfusion.

## Claims

**1.** Compounds of formula (I) :

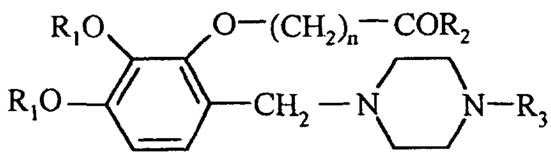

(I)

wherein:

$R_1$ represents a linear or branched ($C_1$-$C_6$)alkyl group,
$R_2$ represents a hydroxy group, a linear or branched ($C_1$-$C_6$)alkoxy group, or an amino group (optionally substituted by one or two linear or branched ($C_1$-$C_6$)alkyl groups),
n represents an integer from 1 to 12 inclusive.
$R_3$ represents :

- a hydrogen atom,
- a linear or branched ($C_1$-$C_{12}$)alkyl group optionally substituted by a phenyl group (itself optionally substituted by one or more identical or different halogen atoms or linear or branched ($C_1$-$C_6$)alkyl, linear or branched ($C_1$-$C_6$)alkoxy, hydroxy or trihalomethyl groups), a ($C_3$-$C_7$)cycloalkyl group, a carboxy group or a ($C_1$-$C_6$)alkoxy-carbonyl group,
- a ($C_3$-$C_7$)cycloalkyl group,
- a pyrimidinyl group optionally substituted by one or two pyrrolidinyl groups, or
- a phenyl group optionally substituted by one or more identical or different halogen atoms or linear or branched ($C_1$-$C_6$)alkyl, linear or branched ($C_1$-$C_6$)alkoxy, hydroxy or trihalomethyl groups,

their optical isomers and addition salts thereof with a pharmaceutically acceptable acid or base.

**2.** Compounds of formula (I) according to claim 1 wherein $R_1$ represents a methyl group.

**3.** Compounds of formula (I) according to claim 1 wherein $R_3$ is an alkyl group substituted by a phenyl group, preferably a benzyl group, a pyrimidinyl group, a phenyl group advantageously substituted by a methoxy group, or a hydrogen atom.

**4.** Compounds of formula (I) according to claim 1 wherein n is an integer from 1 to 7.

**5.** Compounds of formula (I) according to claim 4 wherein n is 6.

**6.** Compounds of formula (I) according to claim 1 wherein $R_2$ represents a hydroxy group.

**7.** Compound of formula (I) according to claim 1 which is 7-{6-[(4-benzyl-1-piperazinyl)methyl]-2,3-dimethoxyphenoxy}heptanoic acid, and addition salts thereof

**8.** Compounds of formula (I) according to claim 1 which are 7-{6-{[4-(pyrimidin-2-yl)-1-piperazinyl])methyl}-2,3-dimethoxyphenoxy}heptanoic acid, 4-{6-[(piperazin-1-yl)-methyl]-2,3-dimethoxyphenoxy}butyric acid, 7-{6-[(4-(2-methoxyphenyl)-1-piperazinyl)-methyl]-2,3-dimethoxyphenoxy}heptanoic acid, 4-{6-[(4-(2-methoxyphenyl)-1-piperazinyl)methyl]-2,3-dimethoxyphenoxy}butyric acid, and addition salts thereof.

**9.** Process for the preparation of compounds of formula (I) according to claim 1,
**characterised in that** there is used as starting material a compound of formula (II) :

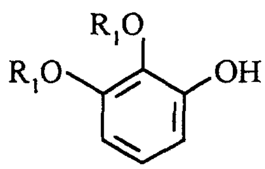

(II)

wherein $R_1$ is as defined for formula (I), which is reacted, in the presence of formaldehyde, with a substituted piperazine of formula (III) :

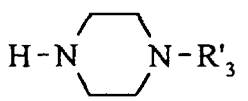

(III)

wherein $R'_3$ is as defined for $R_3$ with the exception of an alkyl group substituted by a carboxy group, to yield a compound of formula (IV) :

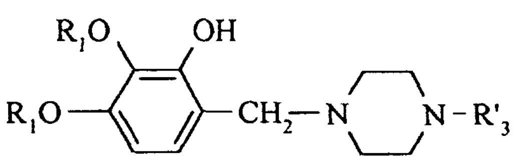

(IV)

wherein $R_1$ and $R'_3$ are as defined hereinbefore, which is reacted with a compound of formula (V) :

$$X\text{-}(CH_2)_n\text{-}COR'_2 \qquad (V)$$

wherein X represents a halogen atom and $R'_2$ represents a linear or branched ($C_1$-$C_6$)alkoxy group, to yield a compound of formula (I/a), a particular case of the compounds of formula (I) :

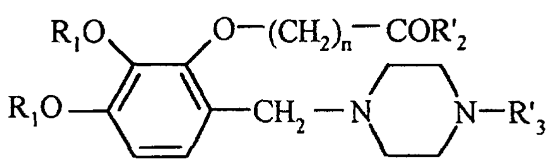

(I/a)

wherein $R_1$, $R'_2$, $R'_3$ and n are as defined hereinbefore,
which compound of formula (I/a) is converted, if desired,

- to the corresponding mono- or di-acid of formula (I/b), a particular case of the compounds of formula (I) :

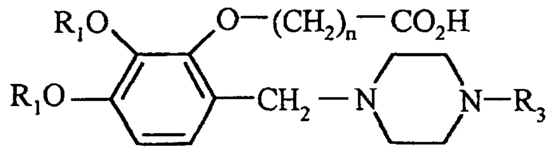

(I/b)

- or to the corresponding amide of formula (I/c) :

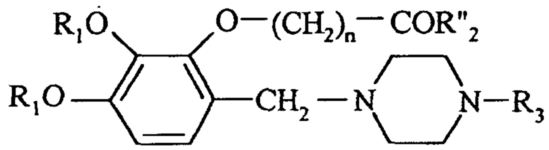

(I/c)

wherein $R_1$, $R_3$ and n are as defined for formula (I) and $R''_2$ represents an amino group optionally substituted by one or two linear or branched ($C_1$-$C_6$)alkyl groups,
which compounds of formulae (I/a), (I/b) and (I/c)
are purified, if necessary, in accordance with a conventional purification technique, are separated, where appropriate, into their isomers, and are converted, if desired, into addition salts with a pharmaceutically acceptable acid or base.

**10.** Pharmaceutical compositions comprising as active ingredient a compound according to any one of claims 1 to 8 in combination with one or more inert, non-toxic, pharmaceutically acceptable excipients or carriers.

**11.** Pharmaceutical compositions according to claim 10 comprising an active ingredient according to any one of claims 1 to 8 for use in the prevention or treatment of acute and chronic cell ischaemia, acute or chronic cerebral, cardiac or peripheral ischaemic accidents, whether followed by reperfusion or not, before any surgical intervention requiring a temporary interruption of blood circulation, in the treatment of chronic neurodegenerative diseases, and in improving the storage of organs intended for transplantation and the resumption of functioning of transplants after reperfusion.